# EUROPEAN PATENT APPLICATION

(11) **EP 3 216 359 A1**
(43) Date of publication of application: **13.09.2017**
(21) Application number: 17171847.1
(22) Date of filing: 19.05.2017
(51) Int. Cl.: A24F 47/00, A61M 15/06, A61M 11/04

(54) **ATOMIZER AND ELECTRONIC CIGARETTE HAVING SAME**

(30) Priority: 27.05.2016 CN 201620493855 U
(71) Applicant: Shenzhen First Union Technology Co., Ltd., Shenzhen, Guangdong 518104 (CN)
(72) Inventor: LI, Yonghai, Shenzhen, Guangdong 518104 (CN); XU, Zhongli, Shenzhen, Guangdong 518104 (CN); HU, Shuyun, Shenzhen, Guangdong 518104 (CN)
(74) Representative: ProI European Patent Attorneys

(57) **Abstract**

An exemplary atomizer includes a housing and an atomizing core arranged in the housing. The housing defines a liquid chamber configured for accommodating tobacco liquid. The atomizing core is configured for atomizing the tobacco liquid to form aerosol. The atomizing core includes a hollow tubular heating body and a liquid conducting element wrapping around the heating body. The liquid conducting element is configured guiding the tobacco liquid in the liquid chamber to the heating body for atomization. The heating body defines a passage for aerosol. The heating body is made of metal. The heating body includes a sidewall defining a plurality of openings.

## Description

### TECHNICAL FIELD

The present invention relates to electronic cigarettes, and particularly to an atomizer and an electronic cigarette using same.

### BACKGROUND ART

In an electronic cigarette, an atomizer includes a heating element and a liquid conducting element in contact with the heating element. Typically, the heating element is a heating wire, and the heating wire is spirally wound around the liquid conducting element. However, a contact surface between the heating wire and the liquid conducting element is small. Accordingly, an amount of aerosol generated by the atomizer is small, rendering some users unsatisfactory.

What are needed, therefore, are an atomizer and an electronic cigarette using same, which can overcome the above shortcomings.

### SUMMARY

An exemplary atomizer includes a housing and an atomizing core arranged in the housing. The housing defines a liquid chamber configured for accommodating tobacco liquid. The atomizing core is configured for atomizing the tobacco liquid to form aerosol. The atomizing core includes a hollow tubular heating body and a liquid conducting element wrapping around the heating body. The liquid conducting element is configured guiding the tobacco liquid in the liquid chamber to the heating body for atomization. The heating body defines a passage for aerosol. The heating body is made of metal. The heating body includes a sidewall defining a plurality of openings.

### BRIEF DESCRIPTION OF THE DRAWINGS

Many aspects of the present disclosure can be better understood with reference to the following drawings. The components in the drawings are not necessarily drawn to scale, the emphasis instead being placed upon clearly illustrating the principles of the present disclosure. Moreover, in the drawings, like reference numerals designate corresponding parts throughout the several views.
FIG. 1 is a partially cut-off view of an atomizer according to an embodiment.
FIG. 2 is a partially cut-off view of an atomizing core of the atomizer of FIG. 1.
FIG. 3 is a heating body according to a first embodiment.
FIG. 4 is a heating body according to a second embodiment.
FIG. 5 is a heating body according to a third embodiment.
FIG. 6 is an electronic cigarette according to a fourth embodiment.

### DETAILED DESCRIPTION

It will be appreciated that for simplicity and clarity of illustration, where appropriate, reference numerals have been repeated among the different figures to indicate corresponding or analogous elements. In addition, numerous specific details are set forth in order to provide a thorough understanding of the embodiments described herein. However, it will be understood by those of ordinary skill in the art that the embodiments described herein can be practiced without these specific details. In other instances, methods, procedures and components have not been described in detail so as not to obscure the related relevant feature being described. Also, the description is not to be considered as limiting the scope of the embodiments described herein. The drawings are not necessarily to scale and the proportions of certain parts have been exaggerated to better illustrate details and features of the present disclosure.

The disclosure is illustrated by way of example and not by way of limitation in the figures of the accompanying drawings in which like references indicate similar elements. It should be noted that references to "an" or "one" embodiment in this disclosure are not necessarily to the same embodiment, and such references mean at least one.

Several definitions that apply throughout this disclosure will now be presented.

The term "outside" refers to a region that is beyond the outermost confines of a physical object. The term "inside" indicates that at least a portion of a region is partially contained within a boundary formed by the object. The term "substantially" is defined to be essentially conforming to the particular dimension, shape or other word that substantially modifies, such that the component need not be exact. For example, substantially cylindrical means that the object resembles a cylinder, but can have one or more deviations from a true cylinder. The term "comprising," when utilized, means "including, but not necessarily limited to"; it specifically indicates open-ended inclusion or membership in the so-described combination, group, series and the like.

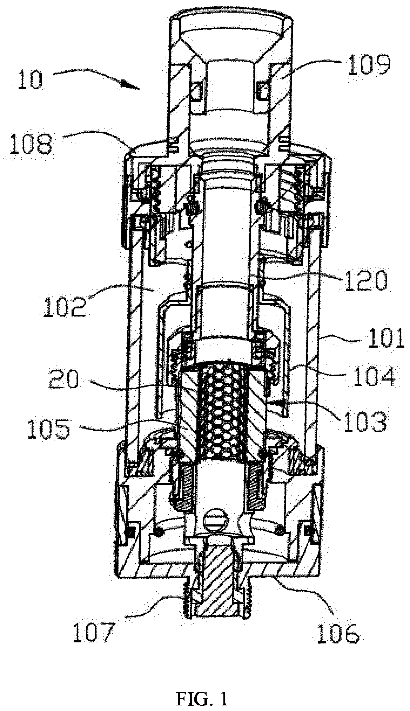

Referring to FIGS. 1-2, an atomizer 10 for an electronic cigarette is shown. The atomizer 10 includes a housing 101, and an atomizing core 103 in the housing 101. The housing 101 defines a liquid chamber 102 for containing tobacco liquid. The atomizing core 103 is configured (i.e., structured and arranged) for atomizing the tobacco liquid to form aerosol. An end cover 108 is provided at an end of the housing 101, and the end cover 108 includes a mouthpiece 109 for inhaling aerosol. The end cover 108 is detachably connected to the housing 101, and is configured for sealing a top opening of the liquid chamber 102. Tobacco liquid can be filled into the liquid chamber 102 when screwing off the end cover 108. An electrical connecting assembly 106 is detachably provided at the other end of the housing 101. The electrical connecting assembly 106 includes a plurality of screw threads 107 for connecting with an external power supply. The atomizing core 103 acquires power via the electrical connecting assembly 106. The atomizing core 103 is detachably arranged on the electrical connecting assembly 106, and is in the liquid chamber 102. When the electrical connecting assembly 106 is detached, the atomizing core 103 can be replaced easily.

The atomizing core 103 includes a hollow tubular heating body 20 and a liquid conducting element 105 wrapping around the heating body 20. The liquid conducting element 105 is configured for guiding the tobacco liquid in the liquid chamber 102 to the heating body 20 for atomization. The heating body 20 defines a passage 202 allowing the aerosol to flow out. The heating body 20 is made of metallic material, and defines a plurality of openings evenly arranged in a sidewall.

In the present embodiment, the heating body 20 is made of metallic material with a certain electrical resistivity and a relatively high strength. Quite usefully, the heating body 20 is made of 316 stainless steel, 304 stainless steel, nickel chromium alloy, or iron-chromium-aluminum alloy. The liquid conducting element 105 may be made of cotton cloth, fiber cotton, or glass fiber or other porous material. The heating body 20 is a metallic tube, and is in fully contact with the liquid conducting element 105, so that the tobacco liquid in the liquid conducting element 105 is heated uniformly.

An air pipe 120 is further provided in the liquid chamber 102. Atop end of the air pipe 120 is connected to the mouthpiece 109, and a bottom end of the air pipe 120 is connected with the atomizing core 103. Since the heating body 20 is arranged in the atomizing core 103, the mouthpiece 109 is connected with the passage 202 of the heating body 20 via the air pipe 120, so that the aerosol in the passage 202 can be expelled via the mouthpiece 109.

A cover body 104 is provided nesting the air pipe 120. A top end of the cover body 104 is connected with the end cover 108, and the cover body 104 is movable together with the end cover 108. When the end cover 108 is detached for filling tobacco liquid, a bottom end of the cover body 104 hermetically abuts against a bottom of the liquid chamber 102 driven by a spring. In this way, the cover body 104 nests the atomizing core 103, and the tobacco liquid in the liquid chamber 102 cannot flow into the atomizing core, thus avoiding liquid leakage during process of injecting tobacco liquid. After the end cover 108 is coupled to the housing 101, the end cover 108 is capable of driving the cover body 104 to move upwards, so that the tobacco liquid in the liquid chamber 102 flows into the atomizing core 103.

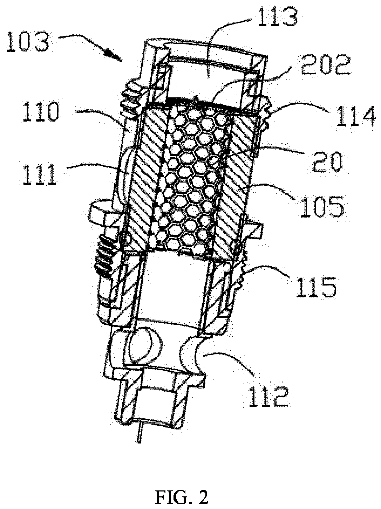

Referring to FIG. 2, the atomizing core 103 further includes a cylindrical shell 110. The heating body 20 and the liquid conducting element 105 are received in the shell 110. The shell 110 defines a plurality of liquid inlets 111 in a sidewall. The tobacco liquid in the liquid chamber 102 can enter the liquid conducting element 105 via the liquid inlets 111 and be conveyed to the heating body 20. The shell 110 includes an air inlet 112 and an air outlet 113 at two opposite ends. The air inlet 112 and the air outlet 113 are in communication with each other via the passage 202 in the heating body 20. The shell 110 includes a plurality of screw threads 114 adjacent to the air outlet 113. The screw threads 114 are configured for connecting with the air pipe 120. The shell 110 further includes a plurality of screw threads 115 adjacent to the air inlet 112. The screw threads 115 are configured for connecting with the electrical connecting assembly 106.

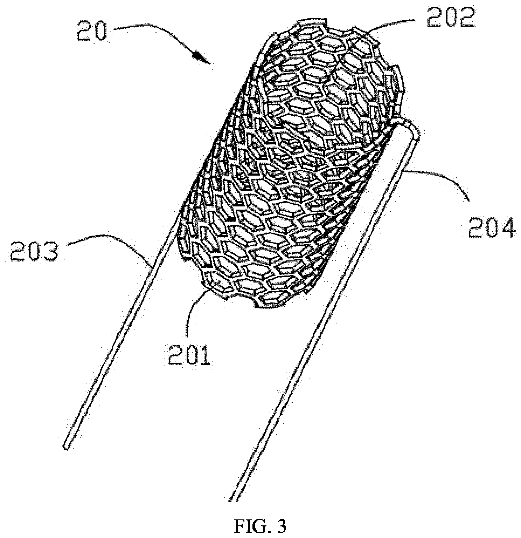

Referring to FIG. 3, a heating body 20 according to a first embodiment is shown. The heating body 20 is formed by a metal foil. First, a plurality of openings are formed in the metal foil by punching, the openings may be hexagon openings 201. Then, the metal foil is rolled to form a tube body by welding. Finally, two electrode parts 202, 203 are formed at two ends of the tube body. The electrode parts 202, 203 are connected to a positive electrode and a negative electrode of the electrical connecting assembly 106, respectively. The heating body 20 can heat uniformly. Further, a contact surface between the heating body 20 and the liquid conducting element 105 is large. Accordingly, an amount of aerosol generated by the heating body 20 is large.

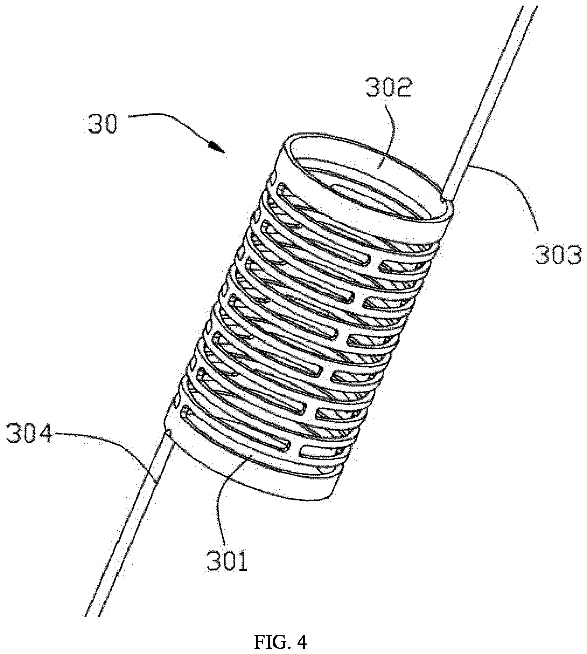

Referring to FIG. 4, a heating body 30 according to a second embodiment is shown. The heating body 30 is made of a metallic tube. The metallic tube defines a passage 302 for aerosol extending through the metallic tube. Two ends of the metallic tube are connected with electrode parts 303, 304. The metallic tube defines a plurality of strip shaped openings 301. The strip shaped openings 301 are not in communication with each other, and may be formed by etching. Quite usefully, the openings 301 extend along a circumferential direction of the sidewall. The openings 301 in two adjacent rows are in a staggered fashion. It is to be understood that, in other embodiments, the openings 301 may extend along an axial direction of the tube body.

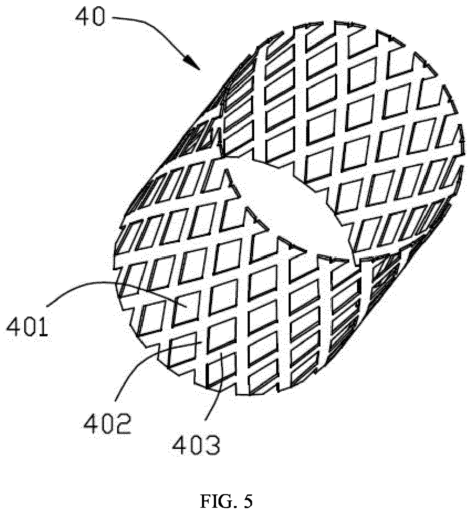

Referring to FIG. 5, a heating body 40 according to a third embodiment is shown, in which electrode parts are omitted. The heating body 40 is formed by knitting a plurality of heating wires or stripe shaped heating plates spirally. Taking the stripe shaped heating plate for an example, the heating body 40 includes a plurality of groups of heating plates 402, 403. The heating plates 402, 403 are crossed with each and knitted together. A plurality of openings 401 are evenly defined in the heating body 40. To achieve a stable structure, an intersecting point between the heating plates 402, 403 may be welded.

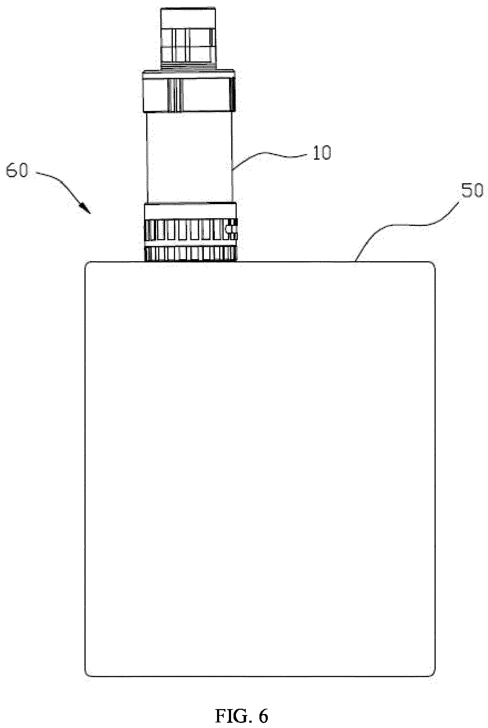

Referring to FIG. 6, an electronic cigarette is shown. The electronic cigarette 60 includes the above atomizer 10 and a power supply 50 connected to the atomizer 10. The electrical connecting assembly 106 of the atomizer 10 is connected to the power supply 50 via screw threads. In this way, the heating body 20, 30 or 40 is electrically connected with the power supply.

It is understood that the above-described embodiments are intended to illustrate rather than limit the disclosure. Variations may be made to the embodiments and methods without departing from the spirit of the disclosure. Accordingly, it is appropriate that the appended claims be construed broadly and in a manner consistent with the scope of the disclosure.

## Claims

1. An atomizer, comprising:
a housing, the housing defining a liquid chamber configured for accommodating tobacco liquid;
an atomizing core arranged in the housing, the atomizing core being configured for atomizing the tobacco liquid to form aerosol;
wherein the atomizing core comprises a hollow tubular heating body and a liquid conducting element wrapping around the heating body, the liquid conducting element is configured guiding the tobacco liquid in the liquid chamber to the heating body for atomization, the heating body defines a passage for aerosol, the heating body is made of metal, the heating body comprises a sidewall defining a plurality of openings.

2. The atomizer according to claim 1, wherein the plurality of openings are evenly arranged in the sidewall.

3. The atomizer according to claim 1, wherein the plurality of the openings are oriented along a circumferential direction of the sidewall.

4. The atomizer according to any of claims 1-3, wherein the heating body further comprises two electrode parts arranged at two ends, and the two electrode parts are configured for connecting to an external power supply.

5. The atomizer according to claim 1, wherein the atomizing core further comprises a shell, the heating body and the liquid conducting element are arranged in the shell, the shell defines a plurality of liquid inlets, and the tobacco liquid in the liquid chamber can flow to the liquid conducting element via the liquid inlets.

6. The atomizer according to claim 1, further comprising a mouthpiece and an air pipe, wherein the mouthpiece is connected to the passage of the heating body via an air pipe.

7. An electronic cigarette comprising:
an atomizer according to any of claims 1-6; and
a power supply connected to the atomizer, the power supply being configured for supplying the atomizer power.
